# EUROPEAN PATENT SPECIFICATION

(11) **EP 0 721 336 B1**
(45) Date of publication and mention of the grant of the patent: **14.07.1999**
(21) Application number: 94924946.0
(22) Date of filing: 30.08.1994
(51) Int. Cl.: A61K 31/425, C07D 277/66, C07D 263/56, A61K 31/42

(54) **BENZAZOLE COMPOUNDS FOR USE IN THERAPY**
BENZAZOLE DERIVATE ALS THERAPEUTISCHE MITTEL
DERIVES DU BENZAZOLE A USAGE THERAPEUTIQUE

(30) Priority: 28.08.1993 GB 9317949
(43) Date of publication of application: 17.07.1996
(73) Proprietor: CANCER RESEARCH CAMPAIGN TECHNOLOGY LIMITED, London NW1Y 4JL (GB)
(72) Inventor: STEVENS, Malcolm Francis Graham, Leicestershire LE12 9XA (GB); McCALL, Carol Jane, Pulloxhill Bedfordshire MK45 5HA (GB); LELIEVELD, Petrus, NL-2751 XR Moerkapelle (NL)
(74) Representative: Skerrett, John Norton Haigh
(86) International application number: GB9401883
(87) International publication number: WO9506469

(56) References cited:
- FR-A- 1 255 312
- FR-A- 2 684 377
- GB-A- 1 080 246
- GB-A- 1 093 355
- GB-A- 2 164 648
- US-A- 2 780 628
- US-A- 4 861 897
- CHEMICAL ABSTRACTS, vol. 71, no. 5, 4 August 1969, Columbus, Ohio, US; abstract no. 22057s, W LEE ET AL 'Induction of increased benzopyrene hydroxylase activity by 2-phenylbenzothiazoles and related compounds' page 323 ; & CANCER RES., vol.28, no.12, 1968 pages 2539 - 2544
- CHEMICAL ABSTRACTS, vol. 113, no. 24, 10 December 1990, Columbus, Ohio, US; abstract no. 221786q, HU SHENGZHI ET AL 'Crystal structures and antitumor activity of 2-(2'pyridyl)benzothiazole and its organotin complex' page 661 ; & INORG. CHIM. ACTA, vol.173, no.1, 1990 pages 1 - 4
- PATENT ABSTRACTS OF JAPAN vol. 10, no. 208 (C-361) 22 July 1986 & JP,A,61 105 975 (TAKEDA CHEM. IND. LTD) 13 March 1986
- PATENT ABSTRACTS OF JAPAN vol. 13, no. 402 (C-633) 6 September 1989 & JP,A,01 146 875 (KANEBO LTD) 8 June 1989
- JOURNAL OF MEDICINAL CHEMISTRY, vol.13, no.5, September 1970, WASHINGTON US pages 1012 - 1013 STIG AKERFELDT 'Studies on the in vivo antiviral effects of benzothiazole derivatives against various influenza a2 srrains'
- PATENT ABSTRACTS OF JAPAN vol. 6, no. 90 (C-104) 27 May 1982 & JP,A,57 021 375 (YAMANOUCHI PHARMCEUT.CO LTD) 4 February 1982
- JOURNAL OF MEDICINAL CHEMISTRY, vol.13, no.4, July 1970, WASHINGTON US pages 697 - 704 THEODORE H. HASKELL ET AL 'Neuramidinase inhibition and viral chemotherapy'

## Description

### Field of the Invention

The present invention relates to certain biologically active benzazole compounds, particularly benzothiazole and benzoxazole compounds, which are able selectively to inhibit proliferation of certain mammalian tumour cells, particularly breast cancer cells, and which are accordingly of interest for use as active chemotherapeutic agents in antitumour therapy, especially in connection with the treatment of breast cancer.

### Summary of the Invention

In one aspect this invention provides, for use in therapy, 2-arylbenzazole compounds that are especially active in inhibiting proliferation of certain breast cancer tumour cells, said compounds being exemplified by 2-(4-aminophenyl)benzothiazole and close analogues or acid addition salts thereof.

More specifically, the 2-arylbenzazole compounds of the present invention are generally compounds having the structural formula I, 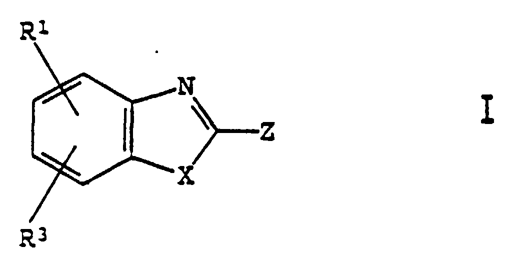 characterised in that
X is S or O;
R¹ and R³ are each independently hydrogen, alkyl, hydroxyl, alkoxy or aralkoxy; and
Z is a group represented by the structural formula II below 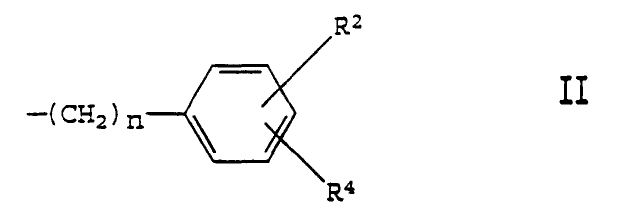 where
   n = 0 or 1,
   R² is hydrogen, NH₂, NO₂, N₃, halogen or an alkyl or substituted alkyl oxysulphonyl group; and
   R⁴ is hydrogen, NO₂, N₃, pyrrolidino, piperidino, morpholino or NR⁵R⁶ where
      R⁵ and R⁶ each represent hydrogen or alkyl;
with the proviso that when R⁴ is hydrogen R² is selected from NH₂, NO₂ and N₃, and when one of R² and R⁴ is hydrogen and the other is -NH₂ neither R¹ nor R³ is 6-alkyl,
and with the further proviso that alkyl groups when present as such in the compound or as a moiety in other groups such as alkoxy are each composed of less than 6 carbon atoms.

The invention also includes compounds that in use can act as precursors or prodrugs and readily break down or be converted, for example by metabolic processes, in the animal body or other biological systems so as to form the particular biologically active compounds hereinbefore specified, especially 2-arylbenzazole compounds in which the aryl group has an amino group substituent. The claims appended hereto should therefore be construed accordingly in determining the scope of the invention.

In at least most preferred embodiments of the invention wherein the benzazole compound is as specified above, at least one of R² and R⁴ will generally be an amino or a substituted amino group, or some other nitrogen-containing group convertible into an amino group. Furthermore, usually, but not necessarily, R⁴ will be para or in the 4-position of the phenyl group.

Particularly preferred embodiments include at least some compounds in which Z can be represented by the structural formula 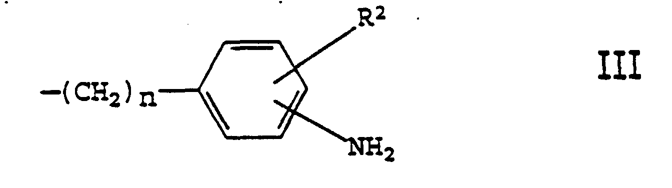 where R² is as previously defined. If R² is halogen, often it will preferably be a 2-Cl group, although in one compound hereinafter listed in accordance with the invention it is a 3-I group.

In some compounds within the scope of the invention, it is also possible for the group -(CH₂)ₙ- in the above structure II to be replaced by an alkenylic group such as -CH=CH-.

Preferred compounds of the invention in accordance with formula I wherein R³ is hydrogen include compounds in which R¹ is alkoxy or benzyloxy, and X is preferably sulphur. More generally, preferred compounds of the invention in accordance with the structural formula I may be further characterised by at least one of the following features:
(a) at least some alkyl groups when present as such or as a moiety in other groups such as alkoxy are methyl or ethyl;
(b) halo substituents, when present, are selected from fluorine and chlorine;
(c) R² is hydrogen or 2-halogen, and R⁴ is amino.

Compounds in accordance with the invention which conform to formula I wherein Z has the structural formula II with n=0, and which are of particular interest, include those compounds where the combination of substituents R¹, R², R³, R⁴ and X is selected from the following combinations:

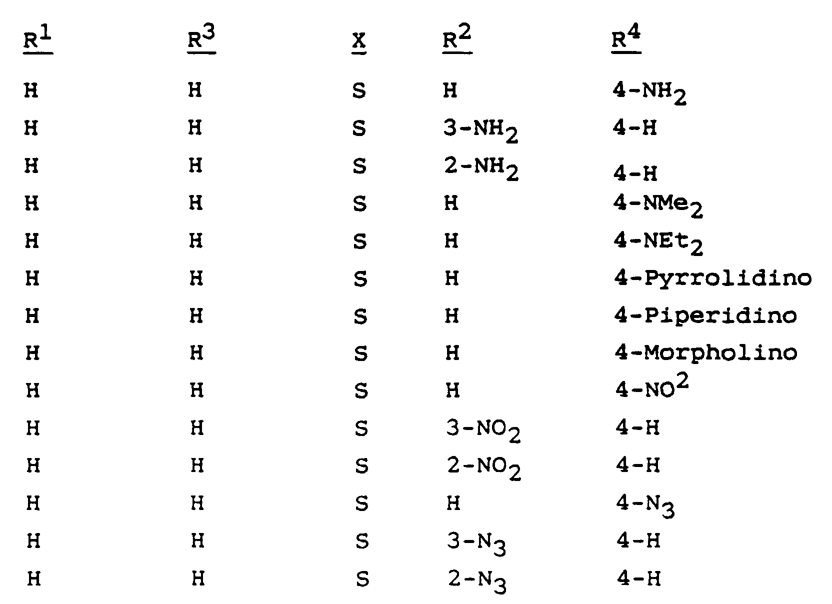

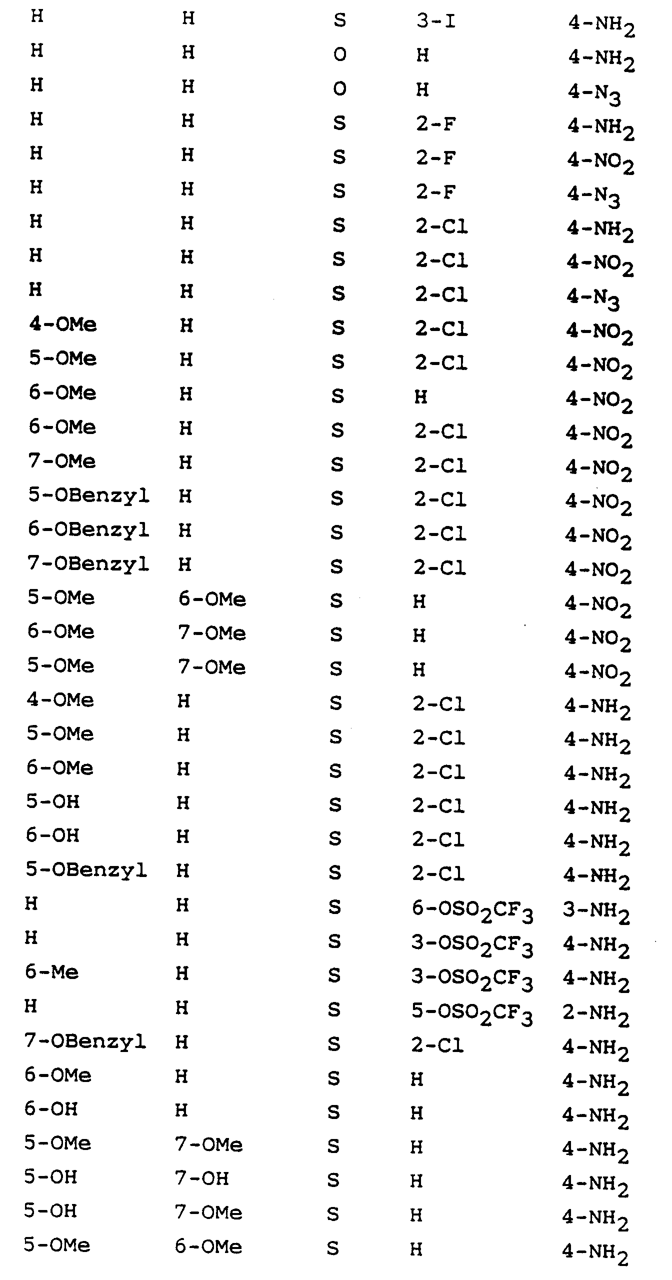

In preferred compounds, as may be apparent from the above list, R¹ and R³ will often both be hydrogen but one or other may alternatively be alkoxy or aralkoxy, e.g. methoxy or benzyloxy, whilst at least one of R² and R⁴ is preferably an, amino or a substituted amino group (e.g. - NMe₂) but may alternatively be nitro, azido, hydroxy, halo, or R² and R⁴ may collectively comprise a combination of such groups substituted at different positions. Other substitution patterns can also be of interest as will be apparent from examples herein disclosed. Although in some cases the nitro and azido substituted compounds themselves exhibit some selective cytotoxic activity, most commonly these derivatives will be used as intermediates for reduction to obtain the corresponding amino compounds. The latter are usually more likely to show a useful level of biological activity.

One especially important compound in the above list is that designated CJM 126 wherein R¹, R² and R³ are each hydrogen, R⁴ is 4-NH₂ and X is sulphur, i.e. 2-(4-aminophenyl)benzothiazole, including pharmaceutically acceptable salts thereof.

Salts of the compounds of formula I in accordance with the invention may include acid addition salts derived from an acid selected from the group comprising: hydrochloric, hydrobromic, sulphuric, nitric, phosphoric, maleic, salicylic, p-toluenesulphonic, tartaric, citric, lactobionic, formic, malonic, pantothenic, succinic, naphthalene-2-sulphonic, benzenesulphonic, methanesulphonic and ethanesulphonic.

The invention also comprises the use of a 2-arylbenzazole compound as specified above for making a medicament or pharmaceutical composition for use in antitumour therapy, especially in the treatment of breast cancer for example.

As hereinafter described, the invention also includes pharmaceutical compositions or preparations, conveniently in unit dosage form, for use in antitumour therapy, especially breast cancer therapy, said compositions or preparations comprising as the active substance a 2-arylbenzothiazole or 2-arylbenzoxazole compound as specified above.

In general, salts of compounds such as CJM 126, e.g. hydrochloride, dihydrochloride, methanesulphonic acid and ethanesulphonic acid addition salts, should be equally effective in inhibiting proliferation of breast tumour cells insofar as such salts will dissociate in water or other aqueous media to provide the active antitumour compound. In practice these salts may be the most preferred compounds for making up acceptable pharmaceutical formulations.

### Development and Testing

The invention has developed from an initial observation made when carrying out certain routine chemical investigations. During these investigations it was found that the arylbenzothiazole compound 2-(4-aminophenyl)benzothiazole, herein designated CJM 126 and sometimes referred to as ptiazammine, had a very surprising and completely unexpected high and selective activity as an antiproliferative agent in respect of cultures of MCF-7 mammary carcinoma cells.

This is illustrated in Tables 1 and 2 at the end of the present description which show in vitro test results obtained in various sets of experiments for the cytotoxic activity of this compound CJM 126 against a range of human tumour cell lines, expressed in terms of IC₅₀ values (concentration or dosage required to reduce cell growth or proliferation by 50%) calculated from dose-effect curves plotted for cultures of the cells in question.

It will be seen from Table 1 that the MCF-7 cell line used in the first series of experiments was about 3000 times more sensitive to CJM 126 than other human cell lines tested. This remarkable result has been reconfirmed on several subsequent occasions, as evidenced for example by the results presented in Table 2, using fresh samples and different strains of the MCF-7 cells in later series of experiments. Thus, selective inhibition has been clearly demonstrated, for example against the following human mammary carcinoma cell lines: MCF 7-wt, MDA 468, SKBR3, ZR 75 and MDA 231 with estimated IC₅₀ values of about 2.9nM, 1.6nM, 25.9nM, 24.7nM, and 17.2nM respectively. Also, a somewhat unusual biphasic dose response relationship was noted. For example, in the first series of experiments referred to above it was found that an initial decrease in cell growth for the MCF-7 cells, which gave the IC₅₀ value of approximately 2 ng/ml, was followed at higher doses by a small increase, and then at even greater doses there was a second decrease corresponding to an IC₅₀ value of about 2 µg/ml. This, biphasic dose response effect has also been found to be consistently reproducible. Thus, in the later series of experiments 30nM and 100nM CJM 126 resulted in maximum growth inhibition of MCF-7 cells and loss of growth arrest, characterised by rising cell numbers, followed exposure to 1µM, 3µM and 10µM. For MCF 7-ADR cells at low concentrations of CJM 126 growth was negligible under the experimental conditions used (seeding density 2.5 x 10² cells per well). However, at higher concentrations of 3 and 10µM, CJM 126 induced proliferation of these MCF cells 7-ADR.

Although certain mammary carcinoma cell lines examined did not give a biphasic dose response following CJM 126 exposure, cell lines derived from tumours of other origins appeared generally to be insensitive to the specific inhibitory properties of CJM 126. No effect was observed, for example, on the growth of A431 cells as a result of exposure to CJM 126 over a range of concentrations (0.1nM-10µM). Estimated IC₅₀ values for some of the other cell lines tested are given in Table 2.

In other in vitro studies it was found that CJM 126 gave an IC₅₀ value of 2.7 x 10⁻⁵ M against P388 cells, and against P388R8/22 (multidrug resistant) cells it gave an IC₅₀ value of 5.2 x 10⁻⁵ M. CJM 126 was also tested against the NCI (National Cancer Institute, U.S.A.) panel of human tumour cell lines which does not contain any breast cancer cell lines, and the compound was found to be essentially non-toxic in these tests.

In carrying out the above in vitro studies, the method used for conducting the cytotoxicity assays was generally substantially as follows:
Cells were maintained in a continuous logarithmic culture in Dulbecco's medium supplemented with 10% fetal calf serum and penicillin (100 IU/ml) and streptomycin (100 µg/ml). The cells were mildly trypsinized for passage and for use in assays. On day zero, 100µl of trypsinized tumour cells (1 x 10⁴/ml) were plated in the wells of 96-well flat-bottom microtiter plates. The plates were incubated for 2 days at 37°C and 5% CO₂ in air to allow the cells to adhere and resume exponential growth prior to the addition of drugs.
The compounds being tested were dissolved in a small volume of DMSO and diluted to the desired concentration with growth medium so that the final concentration of DMSO did not exceed 0.25%. On day two 50µl of the highest drug concentration was added to the wells of column 12 and from there serially diluted 3-fold to column 1 by serial transfer of 50µl using an 8-channel micropipette. The final volume of column 1 was adjusted to 100µl. No additions were made to the wells of rows A and B, which served as controls. The plates were further incubated for 5 days at 37°C and 5% CO₂ in air. Each compound was tested in duplicate.
On day 7 the test was terminated by the addition of 100µl saline containing 0.002% w/v propidium iodide (Sigma), 0.3% drawing ink (Staedtler "Marsmatic 745" - Trade Mark) and 0.5% Triton X-100. The plates were kept at 4°C overnight before reading on an inverted microscope equipped with an automated scanning stage. Fluorescence intensity was measured in arbitrary units by a photomultiplier. An HP-87 computer controlled the movement of the stage and also collected and processed the data from the multiplier.
For each compound tested a dose-response curve was obtained and the IC₅₀ value (the drug concentration at 50% inhibition of cell growth) was calculated.

The remarkably specific effects and activity of CJM 126 noted against human MCF-7 cells in vitro has been found subsequently to extend to in vivo tests and also to a broad range of analogues of CJM 126, as illustrated for example in Tables 3, 4 and 5.

Table 3 presents the results, including estimated IC₅₀ values, of cytotoxicity and proliferation or growth stimulation tests carried out in vitro for different breast cancer cell lines, including MCF7-wt, MDA 468 and MCF 7-ADR, in respect of compound CJM 126 and a range of analogues of CJM 126 identified by reference codes in the first column. The structures of these analogues will be apparent from the subsequent description herein.

The results of some of the in vivo tests carried out, and a comparison of the activity of CJM 126 with that of Mitoxantrone, are set out in Table 4. The results of similar tests carried out to evaluate the activity of CJM 126, and also of a close analogue thereof designated CJM 129, against BO (T61) human mammary carcinomas implanted in NMRI-nu/nu female mice are shown in Table 4 together with results obtained using other antitumour drugs for comparison. The results marked * are all statistically significant (p<0.05). As will be seen, CJM 126 had significant activity against MCF-7 and BO mammary tumours and there were no signs of severe toxicity following administration of a single i.p. injection of 120 mg/kg CJM 126 (Table 4). Additionally, the relative BO tumour volume was lower following daily treatment of 1mg/kg compared with 10 or 100mg/kg/day (Table 5).

The effect of CJM 126 (0.1, 0.01 and 0.001mg/kg) has also been investigated in nude mice bearing human MDA 468 xenograft tumours. The biphasic response obtained in vitro following exposure of MDA 468 cultures to CJM 126 was also observed in vivo when comparable CJM 126 doses were administered. Preliminary data indicate maximum inhibition to tumour growth following daily treatment of the minimum concentration tested, as can be seen from the summary of results presented in Table 6.

Although high concentrations of CJM 126 were found actually to stimulate cell proliferation in some cases, experiments have demonstrated that a continued exposure to CJM 126 is absolutely essential to maintain this stimulation of growth effect which is observed, for example, in the 2nd phase of the MCF 7-wt response. Removal of CJM 126 led not merely to loss of proliferative potential but also to a decline in cell numbers. In contrast, it was found that cell growth following exposure to nM CJM 126 concentrations (phase 1) remained arrested after drug removal and an observed IC₅₀ value of 2.18nM was not significantly affected.

In these in vitro assays, it was noted that serum factors appear to play a significant role in the 2nd phase of the CJM 126 dose response. Thus, in the presence of 1% FCS, no proliferation of colonies was observed following exposure to 3, 10, 30µM CJM 126, and growth inhibition was maintained. Moreover, enhanced potency was detected with estimated IC₅₀ values in the picomolar range.

In other experiments it was established that growth of MCF 7-wt cell cultures supplemented with 1% FCS was stimulated 240%, 346% and 397% by 5, 50 and 100ng/ml EGF respectively, but CJM 126 was able to reverse this effect and exquisitely arrest the growth of these cells. However, the dose response profiles and IC₅₀ values did not differ significantly from MCF 7-wt cultures supplemented by 1% FCS in the absence of EGF. It was also noted that the presence of EGF was unable to rescue the 2nd phase of the dose response, and inhibition of growth was greater than 85% for all concentrations of CJM 126 between and including 1nM-10µM. Similarly, in MCF 7-wt cultures supplemented with 10% FCS, the dose response to CJM 126 was not significantly altered by inclusion of EGF (5, 50 and 100ng/ml) in experimental media.

It was also observed that CJM 126 (1µM, 10µM and 100µM) showed no ability to displace iodinated EGF from EGFR. In addition, Western blot analyses utilizing an anti-phosphotyrosine primary antibody demonstrated no apparent inhibition of unstimulated or of EGF stimulated EGFR tyrosine kinase activity, and at present the mode of action of the CJM 126 compound in these biological systems is not known.

In the course of studying the in vivo activity it was found that the lethal dose value LD₅₀ of CJM 126, i.e. the dose that was lethal to 50% of the animals tested, was about 125mg/kg in male DBA/2 mice when administered as a single i.p. dose. With daily administration over 3 consecutive days the LD₅₀ dose was >31mg/kg/day.

### Preparative Methods

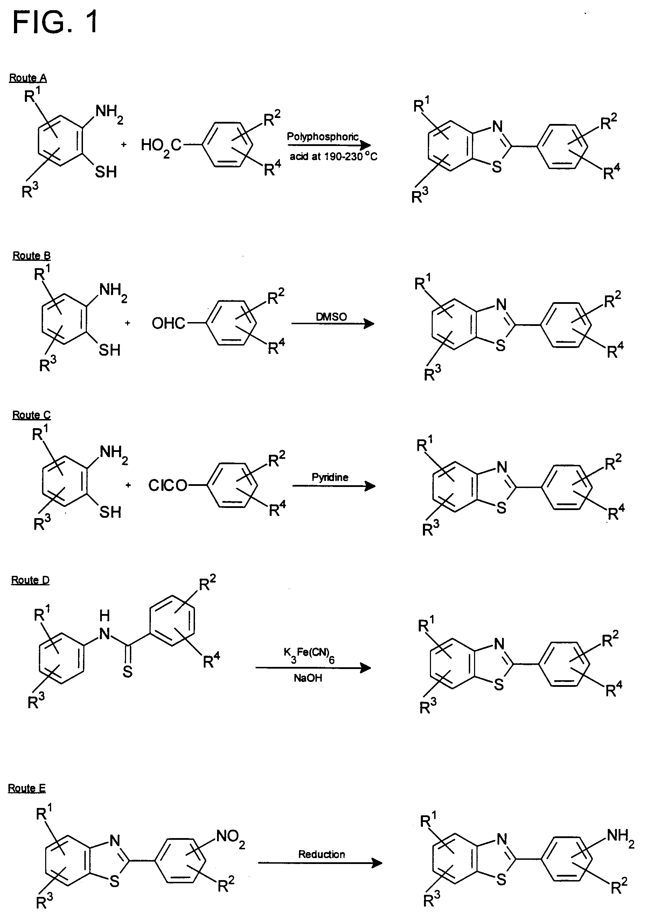

In most cases the arylbenzazole compounds of the present invention can readily be synthesised by various routes from easily available starting materials, and by way of example, several such general synthetic routes, designated Route A, Route B, Route C, and Route D, are illustrated in Figure 1 of the accompanying drawings in relation specifically to arylbenzothiazole compounds. A reduction scheme for converting a nitro substituent of an arylbenzothiazole compound into an amino substituent is also depicted as Route E. Such nitro compounds are often prepared for use as intermediates in producing the corresponding amino compounds which usually may be expected to possess greater cytotoxic activity.
Some of the arylbenzazole compounds of the present invention are also known compounds per se that are already commercially available.
In the general method for Route A, which is also applicable to the synthesis of corresponding benzoxazole compounds, typically a mixture of the 2-aminothiophenol (0.05 Mol.) (or the 2-aminophenyl) and the appropriate benzoic acid derivative (0.05 Mol.), together with polyphosphoric acid (85g), is heated at 190-230°C for 4 hours, cooled and poured into a mixture of 10% aqueous sodium bicarbonate (1000ml) and ice. The solid product may then be collected, washed with water and recrystallized.
In the general method for Route B, typically a mixture of 2-aminothiophenol (0.05 Mol.), the appropriate benzaldehyde (0.05 Mol.) and dimethylsulphoxide (30 ml) is heated to 180°C for 15 minutes, cooled and diluted with water (200 ml). The precipitate is then collected, washed with water and crystallised.
In the general method for Route C, assuming for example that R² is a nitro group NO₂, a solution of the 2-aminothiophenol (0.05 Mol.) in pyridine (50 ml) is added slowly to a mixture of the appropriate nitrobenzoyl chloride (0.05 Mol.) also in pyridine (50 ml) at 25°C. The reaction is exothermic and is cooled in an ice-bath. The mixture may then be diluted with water (200 ml) and the products are collected and washed with water.
In the general method for Route D, in a typical procedure the appropriate substituted thiobenzanilide (1 Mol. equiv.) is finely powdered and mixed with a little ethanol to form a wet paste. A 30% w/v solution of aqueous sodium hydroxide (8 Mol. equiv.) is added and diluted with water to form a suspension/solution of the thiobenzanilide in 10% w/v aqueous sodium hydroxide. Aliquots of this suspension/solution are then introduced dropwise at one minute intervals into a stirred solution of potassium ferricyanide (4 Mol. equiv.) in water at 80-90°C. The reaction mixture is heated for a further 30 minutes, then cooled. The 2-arylbenzothiazole products are collected, washed with water and crystallised.
Where R² of the 2-arylbenzazole compound synthesised by any of the above routes (or by any other route) is a nitro group NO₂, this may generally be reduced and converted into the corresponding amine as follows (Route E):
   A mixture of the 2-(nitrophenyl)benzazole compound in question (0.05 Mol,) and stannous chloride dihydrate (0.25 Mol.) in absolute ethanol (200 ml) is stirred and refluxed under nitrogen for 1 to 4 hours. The ethanol is then removed under reduced pressure and the residue is dissolved in ethyl acetate (4 x 100ml). The combined organic phases are next shaken with excess aqueous sodium hydroxide to liberate the free amine bases and dissolve the tin residues. The separated organic phase is washed with water, dried (magnesium sulphate) and the solvent is evaporated. Finally, the products are then crystallised.

### EXAMPLES

The preparation of a number of particular compounds which are considered to be of interest for use as active therapeutic substances to inhibit proliferation of at least certain breast cancer cells and which provide examples of preferred embodiments of the invention, including CJM 126 and also analogues thereof, will now be described in more detail. The compound reference codes used in Table 3 are also quoted where applicable. It should be understood, however, that these specific examples are not intended to be construed in any way as a limitation in the scope of the invention.

### Example I

### 2-(4-aminophenyl)benzothiazole (Compound CJM 126)

(a) In one particular example, 2-(4-aminophenyl)benzothiazole (Compound CJM 126) was prepared in 57% yield from 2-aminothiophenol and 4-aminobenzoic acid using synthetic Route A, the final product being crystallised from methanol as pale yellow needle crystals having a melting point 155-157°C (aqueous solubility about 3.8µg/ml).
(b) In another preparative example, the same compound 2-(4-aminophenyl)benzothiazole was prepared in 73% yield, as beige needles (m.p. 155-156°C) crystallised from methanol, by reduction with stannous chloride hydrate of 2-(4-nitrophenyl)benzothiazole. The latter was first obtained (as yellow crystals, m.p. 229-231°C, crystallised from methanol or dimethyl formamide) either via Route C (71% yield) from 2-aminothiophenol and 4-nitrobenzoyl chloride, or via Route D (10% yield) from the corresponding thiobenzanilide and potassium ferricyanide.

### Example IA

### Ethanesulphonic acid salt of 2-(4-aminophenyl)benzothiazole (Compound 93003)

To prepare the ethanesulphonic acid salt, 2-(4-aminophenyl)benzothiazole (0.66g) in ethyl acetate (100 ml) at 25°C was treated with ethanesulphonic acid (0.338g). The solid was collected and washed with ethyl acetate (3 x 50 ml) followed by diethyl ether (3 x 50 ml). The ethanesulphonic acid salt (0.89g., yield 90%) had m.p. 211-213°C.

### Example IB

### Dihydrochloride salt of 2-(4-aminophenyl)benzothiazole

To prepare the dihydrochloride salt, 2-(4-aminophenyl)benzothiazole (1.5g) was dissolved in ethyl acetate (250 ml) and a stream of dry hydrogen chloride was passed through the solution for 20 minutes. The yellow dihydrochloride salt was collected and washed with ethyl acetate followed by ether. The dihydrochloride (1.61g) had a m.p. 267-269°C.

### Example IC

### 2-(4-Aminophenyl)benzothiazole methanesulphonic acid salt

To a solution of 2-(4-aminophenyl)benzothiazole (0.5g, 2.21mmol) in ethyl acetate (65ml) was added dropwise methanesulphonic acid (0.215g, 2.21mmol) at room temperature. The reaction mixture was stirred for 30 minutes. The product was collected and washed with hot ethyl acetate to give a pale yellow powder (0.67g, 94%), m.p. 261-262°C; νₘₐₓ/cm⁻¹ 3422, 2880, 2633, 1598, 1487, 1322, 1220, 1149, 1043, 967, 780, 755, 561; δ_{H}(DMSO-d₆) 8.10(1H, d, *J*7.9, 4-H), 8.00(3H, m, 7,2',6'-H), 7.52(1H, t, *J*7.3, 5-H), 7.42(1H, t, *J*7.5, 6-H), 7.06(2H, d, *J*8.5, 3',5'-H), 5.72(3H, br s, NH₃+), 2.41(3H, s, CH₃).
In vitro assay of this compound gave IC₅₀ values of 0.0007µM against MCF 7-wt cells and 0.0024µM against MDA 468 cells, indicating a very high level of selective cytotoxic activity.

### Example II

### 2-(2-Chloro-4-nitrophenyl)-4-methoxybenzothiazole

This compound was prepared from 2-chloro-4-nitro-2'-methoxythiobenzanilide according to the general procedure of synthetic Route D. A yellow solid formed was immediately collected by filtration when 2-chloro-4-nitro-2'-methoxythiobenzanilide was added to the solution of potassium ferricyanide. The crude product was recrystallised twice from methanol to give yellow crystals (46%), m.p. 187-188°C; νₘₐₓ/cm⁻¹ 3432, 3097, 2836, 1570, 1521, 1476, 1385, 1347, 1277, 1262, 1041, 890, 773, 743; δ_{H}(CDCl₃) 8.60(1H, d, *J*8.8, 6'-H), 8.37(1H, d, *J*2.3, 3'-H),8.19(1H, dd, *J*2.3, 8.7, 5'-H), 7.55(1H, d, *J*8.1, 7-H), 7.43(1H, t, *J*8.1₁, 6-H), 6.97(1H, d, *J*7.9, 5-H).

### Example III

### 5-Benzyloxy-2-(2-chloro-4-nitrophenyl)benzothiazole

This compound was prepared from 2-chloro-4-nitro-3'-benzyloxythiobenzanilide, again according to the general procedure of synthetic Route D. The crude reaction product was separated into three fractions by flash column chromatography on silica using EtOAc-hexane-chloroform (1:6:1) as the eluate. The first fraction (52%) was 5-benzyloxy-2-(2-chloro-4-nitrophenyl)benzothiazole, m.p. 156-157°C; νₘₐₓ/cm⁻¹ 3449, 1603, 1521, 1342, 1277, 1181, 1055, 829, 737; δ_{H} (CDCl₃) 8.60(1H, d, *J*8.8, 6'-H), 8.45(1H, d, *J*2.2, 3'-H), 8.27(1H, dd, *J*2.3, 8.8 5'-H), 7.88(1H, d, *J*8.9, 7-H), 7.72(1H, d, *J*2.4, 4-H), 7.55-7.38(5H, m, Ph-H), 7.26(1H, dd, *J*2.4, 8.9, 6-H), 5.23(2H, s, CH₂O).

### Example IV

### 5,7-Dimethoxy-2-(4-nitrophenyl)benzothiazole

This compound was prepared from 4-nitro-3',5'-dimethoxythiobenzanilide, also according to the general procedure of Route D. Recrystallisation from ethanol-EtOAc gave a yellow powder (60%), m.p. 238-239°C; νₘₐₓ/cm⁻¹ 3442, 2949, 1605, 1579, 1528, 1428, 1352, 1312, 1155, 1126, 853, 820, 688; δ_{H}(CDCl₃) 8.37(2H, d, *J',5'-H), 8.26(2H, d, J*9.0, 2',6'-H), 7.24(1H, d, *J*2.0, 4-H), 6.58(1H, d, *J*2.0, 6-H), 4.01(3H, s, 7-OCH₃), 3.95(3H, s, 5-OCH₃); *m/z* 316(M⁺), 270(M-OCH₃ and -CH₃).

### Example V

### 6-Methoxy-2-(4-nitrophenyl)benzothiazole

This compound was prepared from 4'-methoxy-4-nitrobenzanilide, again according to the general procedure of Route D. The crude product was purified by flash column chromatography using EtOAc-hexane (1:3) as the eluate to give the title compound (62%), m.p. 216-217°C; νₘₐₓ/cm⁻¹ 3448, 1593, 1518, 1342, 1315, 1265, 1219, 1066, 849; δ_{H} (CDCl₃) 8.34(2H, d, *J*9.1, 3',5'-H), 8.21(2H, d, *J*9.1, 2',6'-H), 8.00(1H, d, *J*9.0, 4-H), 7.38(1H, d, *J*2.5, 7-H), 7.15(1H, dd, *J*2.6, 9.0, 5-H), 1.58(3H, s, CH₃), m/z 289 (M⁺), 271(M-CH₃).

### Example VI

### 2-(4-amino-2-chlorophenyl)-4-methoxybenzothiazole (Compound 93005)

This amine was obtained in 77% yield by reduction of 2-(2-Chloro-4-nitrophenyl)-4-methoxybenzothiazole according to the general procedure of synthetic Route E. Characterisation of the product was as follows:
mp. 148-150°C; νₘₐₓ/cm⁻¹ 3460, 3316, 3205, 2962, 1626, 1602, 1567, 1474, 1441, 1414, 1335, 1257, 1043, 772, 741; δ_{H}(CDCl₃) 8.19 (1H, d, *J*8.6, 6'-H), 7.49 (1H, d, *J*8.0, 7-H), 7.31 (1H, t, *J*8.0, 6-H), 6.91 (d, *J*7.8, 5-H), 6.76 (1H, d, *J*2.3, 3'-H), 6.64 (1H, dd, *J*2.3, 8.6, 5,-H), 4.07-4.04 (5H, m, OCH₃, NH₂).

### Example VII

### 2-(4-Amino-2-chlorophenyl)-5-benzyloxybenzothiazole (Compound DF161)

This amine was obtained by reduction of 5-benzyloxy-2-(2-chloro-4-nitrophenyl)benzothiazole, again according to the general procedure of Route E. The crude product was chromatographed on silica using EtOAc-hexane (1:1) as the eluate to give 2-(4-amino-2-chlorophenyl)-5-benzyloxybenzothiazole (90%), m.p. 119-121°C; νₘₐₓ/cm⁻¹ 3456, 3380, 1629, 1599, 1443, 1260, 1171, 1051, 802; δ_{H}(CDCl₃) 8.12(1H, d, *J*8.6, 6'-H), 7.79(1H, d, *J*8.7, 7-H), 7.65(1H, d, *J*2.4, 4-H), 7.54-7.36(5H, m, Ph-H), 7.14(1H, dd, *J*2.5, 8.8, 6-H), 6.81(1H, *J*2.3, 3'-H), 6.69(1H, dd, *J*2.3, 8.6, 5'-H), 5.20(2H, s, CH₂O), 3.77(2H, s,NH₂).

### Example VIII

### 2-(4-Aminophenyl)-5,7-dimethoxybenzothiazole (Compound DF1620)

This amine was formed by reduction of 5,7-dimethoxy-2-(4-nitrophenyl)benzothiazole, also according to the general procedure of Route E. The crude product was chromatographed on silica using EtOAc-hexane (1:1) as the eluate to give 2-(4-aminophenyl)-5,7-dimethoxybenzothiazole (89%), m.p. 150-152°C; vₘₐₓ/cm⁻¹ 3417, 3326, 3203, 3000, 1599, 1580, 1485, 1415, 1219, 1150, 1124, 1035, 824, 807, 631; δ_{H}(CDCl₃) 7.91(2H, d, *J*8.6, 2',6'-H), 7.16(1H, d, *J*2.0, 4-H), 7.75(2H, d, *J*2.0, 6-H), 4.03(2H, s, NH₂), 3.96(3H, s, 7-OCH₃), 3.91(3H, s, 5-OCH₃).

### Example IX

### 2-(4-Aminophenyl)-6-methoxybenzothiazole (Compound 93002)

This amine was formed by reduction of 6-methoxy-2-(4-nitrophenyl)benzothiaxzole, also according to the general procedure of Route E. The crude product was chromatographed on silica using EtOAc-hexane (1:1) as the eluate to give 2-(4-aminophenyl)-6-methoxybenzothiazole (92%), m.p. 191-193°C; νₘₐₓ/cm⁻¹ 3454, 1626, 1605, 1465, 1436, 1264, 1222, 825; δ_{H} (DMSO-d₆) 7.79(1H, d, *J*8.9, 4-H), 7.70(2H, d, *J*8,6, 2',6'-H), 7.62(1H, d, *J*2.5, 7-H), 7.05(1H, dd, *J*2.6, 8.9, 5-H), 6.66(2H, d, *J*8.6, 3',5'-H), 5.84(2H, s, NH₂), 3.84(3H, s, OCH₃); *m/z* 256 (M⁺), 241 (MOCH₃).

### Example X

### 2-(4-Amino-2-Chlorophenyl)benzothiazole (Compound 93004)

This amine was formed by reduction of 2-(2-chloro-4-nitrophenyl)benzothiazole, again according to the general procedure of Route E. Recrystallisation from methanol gave yellow crystals (93%), m.p. 92-94°C; νₘₐₓ/cm⁻¹ 3455, 3299, 3194, 1630, 1620, 1432, 1312, 1262, 1050, 847, 757, 729, 699, 626; δ_{H}(CDCl₃) 8.10 (1H, d, *J*8.6, 6'-H), 8.04 (1H, d, *J*7.7, 4-H), 7.89 (1H, d, *J*7.9, 7-H), 7.47 (1H, dt, *J*1.3, 7.2, 5-H), 7.36 (1H, dt, *J*1.2, 7.3, 6-H), 6.78 (1H, d, *J*2.4, 3'-H), 6.66 (1H, dd, *J*2.4, 8.6, 5'-H), 4.02 (2H, s, NH₂).

### Example XI

### 2-(4-Aminophenyl)-5,7-dihydroxybenzothiazole (Compound DF162Eb)

To a stirred solution of 2-(4-aminophenyl)-5,7-dimethoxybenzothiazole (0.24 g, 0.838 mmol), obtained from Example VIII, in dry dichloromethane (15ml) under nitrogen and at -70°C, was added dropwise boron tribromide (1.0 M solution) in dichloromethane (5.9 ml) over 30 minutes. The mixture was kept stirred at -70°C for a further 1 hour, then allowed to warm slowly to room temperature and stirred overnight. The mixture was then again cooled to -70°C and methanol was added dropwise until no further reaction was observed. It was then poured into 8% (W/V) aqueous sodium hydroxide (50ml). After acidification with 5M hydrochloric acid to pH7, the mixture was extracted with dichloromethane-methanol (4:1) (3 x 80 ml). The combined extracts were dried (MgSO₄) and evaporated under reduced pressure. The crude product was then separated into two fractions by flash column chromatography on silica using EtOAc-hexane (3:2) as the eluant. The second fraction (62%) was the required 2-(4-aminophenyl)-5,7-dihydroxybenzothiazole, m.p. 294°C (dec); νₘₐₓ/cm⁻¹ 3438, 3342, 3216, 1603, 1482, 1456, 1434, 1295, 1159, 1093, 829; δ_{H} (DMSO-d₆) 10.30(1H, s, 7-OH), 9.43(1H, s, 5-OH), 7.70(2H, d, *J*8.6, 2',6'-H), 6.74(1H, d, *J*2.0, 4-H), 6.64(2H, d, *J*8.6, 3',5'-H), 6.34(1H, d, *J*2.0, 6-H), 5.84(2H, s, NH₂).

### Example XII

### 2-(4-Aminophenyl)-6-hydroxybenzothiazole

This compound was obtained by demethylation with boron tribromide from 2-(4-aminophenyl)-6-methoxybenzothiazole (see Example IX) in a manner similar to that described above for Example XI. The crude product was chromatographed on silica using EtOAc-hexane (1:1) as the eluate to give the title compound (89%), m.p. 262-263°C; νₘₐₓ/cm⁻¹ 3487, 3388, 1620, 1460, 1289, 1238, 1175, 830; δ_{H} (DMSO-d₆) 9.71(1H, s, OH), 7.72,7.66(3H, m, 4.2',6'-H), 7.32(1H, d, *J*2.3, 7-H), 6.91(1H, dd, *J*2.4, 8.7, 5-H), 6.65(2H, d, *J*8.6, 3',5'-H), 5.80(2H, s, NH₂); *m/z* 242 (M₊).

### Example XIII

### 2-(4-Aminophenyl)benzoxazole (Compound DF140)

This provides an example of a benzaxazole instead of a benzothiazole. In accordance with synthetic Route A, a mixture of 2-aminophenol (1.5g, 0.0136mol) and 4-aminobenzoic acid (1.885g, 0.0136mol) in polyphosphoric acid (20g) was heated at about 190°C for 4 hours, then cooled and poured into 10% aqueous sodium bicarbonate (400 ml). The product was collected by filtration, washed with water and recrystallised from methanol-H₂O (10:1) to give small pale yellow crystals (1.76g, 62%), m.p. 176-178°C (lit 185°C); νₘₐₓ/cm⁻¹ 3472, 3322, 3186, 1614, 1500, 1454, 1312, 1242, 1170, 1066, 830, 742; δ_{H}(DMSO-d₆) 7.87(2H, d, *J*8.6, 2', 6'-H), 7.69-7.64(2H, m, 5, 6-H), 7.36-7.28(2H, m, 4, 7-H), 6.70(2H, d, *J*8.6, 3', 5'-H), 6.02 (2H, s, NH₂).

### Example XIV

### 2-(3-Amino-6-trifluoromethylsulphonyloxyphenyl)benzothiazole

2-(3-Azidophenyl)benzothiazole (1g) was added in small portions (5 x 0.2g) to a mixture of trifluoromethanesulphonic acid (4 ml), trifluoroacetic acid (5 ml) and trifluoromethylacetic anhydride (1 ml) at 0°C. After evolution of nitrogen ceased, the mixture was stirred at 20°C for 18 hours, basified with aqueous ammonia and the products extracted into ethyl acetate. The organic layer was washed with water, dried (MgSO₄) and evaporated to give a gum which was separated on silica with hexane-ethyl acetate (6:4) as eluent. The product obtained was the title compound (70% yield). (Found: M⁺ 374. C₁₄H₉F₃N₂O₃S₂ requires MW 374).
An in vitro assay of this compound against MCF-7 (wild type) cells gave an IC₅₀ value of 50nM which again indicates a useful level of activity.

### Example XV

### 2-(4-Amino-3-trifluoromethylsulphonyloxyphenyl)benzothiazole

This compound was prepared in a similar way to the compound of Example XIV from 2-(4-azidophenyl)benzothiazole. The yield was 12%, (Found: M⁺ 374. C₁₄H₉F₃N₂O₃S₂ requires MW 374).

### Example XVI

### 2-(4-Amino-3-trifluoromethylsulphonyloxyphenyl)-6-methylbenzothiazole

This compound was prepared in a similar way to the compound of Example XIV from 2-(4-azidophenyl)-6-methyl-benzothiazole. (Found: M⁺ 388. C₁₅H₁₁F₃N₂O₃S₂ requires MW 386).

### Example XVII

### 2-(2-Amino-5-trifluoromethylsulphonyloxyphenyl)benzothiazole

This compound was prepared in a similar way to the compound of Example XIV from 2-(2-azidophenyl)benzothiazole (Found: M⁺ 374. C₁₄H₉F₃N₂O₃S₂ requires MW 374).

Other analogue compounds or derivatives of interest that have been prepared using Route A comprise
2-(4-Dimethylaminophenyl)benzothiazole
2-(4-Diethylaminophenyl)benzothiazole
2-(2-Aminophenyl)benzothiazole
2-(2-Fluorophenyl)benzothiazole
2-(4-Aminobenzyl)benzothiazole
and using Route B comprise
2-[4-(Pyrrolidin-1-yl)phenyl]benzothiazole (Compound 93006)
and using Route C comprise
2-(3-Nitrophenyl)benzothiazole 2-(2-Chloro-4-nitrophenyl)benzothiazole
and using Route D comprise

2-(2-Chloro-4-nitrophenyl)-6-methoxybenzothiazole
2-(2-Chloro-4-nitrophenyl)-7-methoxybenzothiazole
and by reduction of the corresponding nitro compound using Route E comprise
2-(3-Aminophenyl)benzothiazole (Compound CJM 129)
2-(4-Amino-2-chlorophenyl)-5-methoxybenzothiazole
2-(4-Amino-2-chlorophenyl)-6-methoxybenzothiazole
2-(4-Amino-2-chlorophenyl)-7-methoxybenzothiazole

Further analogues or derivatives of interest that have been prepared include
2-(4-Azidophenyl)benzothiazole
2-[4-(Morpholin-4-yl)phenyl]benzothiazole (Compound 93008)
2-[4-(Piperidin-1-yl)phenyl]benzothiazole (93007)

Commercially available analogue compounds that have been tested and considered to be of interest include 2-(4-aminophenyl)-6-methylbenzothiazole for which in vitro assays against MCF 7-wt cells gave an IC₅₀ value of 0.38µM and against MDA 468 cells an IC₅₀ value of 0.4µM.

It will be appreciated that some of the above listed compounds with the more complex molecular structures, for example DF68D and 126-126, may be expected to break down or to be metabolised after administration to a mammal so as to form a simpler 2-arylbenzazole compound that, in use, is the main biologically active component. Also, many of the compounds with nitrogen-containing substituents, such as azido groups and nitro groups, may similarly be converted, in use, within the body to a corresponding active amino compound.

### Therapeutic Use

As already indicated, the compounds of this invention have been found to inhibit tumour cell proliferation and to have significant selective antitumour activity, especially in respect of breast cancers. Antitumour activity is evidenced for example by reduction of tumour cell number in mammals bearing breast cancer tumours and a consequent increase in survival time as compared to a control provided by animals which are untreated. Antitumour activity is further evidenced by measurable reduction in the size of solid tumours following treatment with the compounds of this invention compared to the tumours of untreated control animals.

Accordingly, as previously stated the compounds of the present invention are of particular interest for the treatment of breast cancer tumours, and the invention further provides a method for the treatment of a patient suffering from breast cancer. For this purpose, an effective non-toxic amount of the active 2-arylbenzazole compound, such as the 2-(4-aminophenyl)benzothiazole compound or an acid addition salt or close analogue thereof as hereinbefore defined, may be suitably administered, orally, parenterally (including subcutaneously, intramuscularly and intravenously), or topically. The administration will generally be carried out repetitively at intervals, for example once or several times a day.

The amount of the active compound which is required in order to be effective as an antitumour agent for treating mammals will of course vary and is ultimately at the discretion of the medical or veterinary practitioner treating the mammal in each particular case. The factors to be considered by such practitioner, e.g. a physician, include the route of administration and pharmaceutical formulation; the mammal's body weight, surface area, age and general condition; and the chemical form of the compound to be administered. However, a suitable effective antitumour dose may be in the range of about 1.0 to about 75 mg/kg bodyweight, preferably in the range of about 5 to 40mg/kg with most suitable doses being for example in the range of 10 to 30mg/kg. In daily treatment for example, the total daily dose may be given as a single dose, multiple doses, e.g. two to six times per day, or by intravenous infusion for any selected duration. For example, for a 75kg mammal, the dose range could be about 75 to 500mg per day, and it is expected that a typical dose would commonly be about 100mg per day. If discrete multiple doses are indicated, treatment might typically be 50mg of the arylbenzazole compound as hereinbefore defined, given 4 times per day in the form of a tablet, capsule, liquid (e.g. syrup) or injection. On account of the biphasic dose response characteristics of many of these compounds, however, care should be taken, particularly in the initial stages of treatment, to ensure that dosage amounts are not too high.

While it may be possible for the active compound of this invention to be administered alone as the raw chemical, it is preferable to present the active compound as a pharmaceutical formulation. Formulations of the present invention, for medical use, comprise the active compound together with one or more pharmaceutically acceptable carriers thereof and, optionally, any other therapeutic ingredients. The carrier(s) must be pharmaceutically acceptable in the sense of being compatible with the other ingredients of the formulation and not deleterious to the recipient thereof.

The present invention therefore further provides a pharmaceutical formulation comprising an arylbenzazole compound as hereinbefore specified (possibly in the form of a free base or a pharmaceutically acceptable acid addition salt) together with a pharmaceutically acceptable carrier thereof.

The formulations include those suitable for oral, rectal, topical and parenteral (including subcutaneous, intramuscular and intravenous) administration.

The formulations may conveniently be presented in unit dosage form and may be prepared by any of the methods well known in the art of pharmacy. All methods include generally the step of bringing the active compound into association with a carrier which constitutes one or more accessory ingredients. Usually, the formulations are prepared by uniformly and intimately bringing the active compound into association with a liquid carrier or with a finely divided solid carrier or with both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught. The active compound may also be presented as a bolus, electuary or paste.

A tablet may be made by compression or moulding, optionally with one or more accessory ingredients. Compressed tablets may be prepared by compressing, in a suitable machine, the active compound in a free-flowing form such as a powder or granules, optionally mixed with a binder, lubricant, inert diluent, surface active or dispersing agent. Moulded tablets may be made by moulding, in a suitable machine, a mixture of the powdered active compound with any suitable carrier.

A syrup may be made by adding the active compound to a concentrated, aqueous solution of a sugar, for example sucrose, to which may be added any accessory ingredient. Such accessory ingredient(s) may include flavourings, an agent to retard crystallisation of the sugar or an agent to increase the solubility of any other ingredient, such as a polyhydric alcohol for example glycerol or sorbitol.

Formulations for rectal administration may be presented as a suppository with a usual carrier such as cocoa butter.

Formulations suitable for parental administration conveniently comprise a sterile aqueous preparation of the active compound which is preferably isotonic with the blood of the recipient.

In addition to the aforementioned ingredients, formulations of this invention, for example ointments, creams and the like, may include one or more accessory ingredient(s) selected from diluents, buffers, flavouring agents, binders, surface active agents, thickeners, lubricants, preservatives (including antioxidants) and the like.

From another aspect, the invention thus also comprises use of a benzazole compound as hereinbefore specified for the manufacture of a medical preparation for the treatment of breast cancer tumours.

**Table 1**

| In vitro cytotoxicity of CJM 126 against human cell lines (1st series of experiments) | |
|---|---|
| Cell Line | IC₅₀(ng/ml) |
| MCF-7 mammary carcinoma | 2 |
| A204 rhabdomyosarcoma | 5943 |
| T24 bladder carcinoma | 16876 |
| WiDr colon carcinoma | 6008 |
| IGR37 melanoma | 23986 |
| HT29 colon carcinoma | 7568 |
| A2780 ovarian carcinoma | 8826 |

**Table 2**

| IC₅₀ values for CJM 126 against human cell lines (2nd series of experiments) | |
|---|---|
| Cell Line | IC₅₀µM |
| MCF 7 mammary (ER+) | 0.003 |
| MCF 7 mammary (ER+) | 0.008 |
| MCF 7-ADR mammary | stimulation > 1µM |
| ZR 75 mammary (ER+) | 0.028 |
| SKBR3 mammary (ER-) | 0.026 |
| MDA 468 mammary (ER-/EGFR+) | 0.0016 |
| MDA 231 mammary (EGFR+/erbB3+) | .017 |
| T47D mammary (ER-) | 44.1 |
| MCF 7-B (ER+) | 62.2 |
| MCF 7-T (ER-) | 12.87 |
| A204 rhabdomyosarcoma | 26 |
| T24 bladder carcinoma | 75 |
| WiDr colon | 26 |
| IGR 37 melanoma | 106 |
| HT 29 colon | 33 |
| A2780 ovarian carcinoma | 39 |
| A2780 ovarian carcinoma | 43 |
| A2780/CisR | 37 |
| HX/62 ovarian carcinoma | 120 |
| SKOV-3 ovarian carcinoma | 64 |
| 41M ovarian carcinoma | 36 |
| 41M/CisP | 40 |
| CH 1 ovarian carcinoma | 22 |
| CH 1/CisR | 33 |

**Table 3**

| Cytotoxicity (IC₅₀) and growth stimulation of analogues of CJM 126 in breast cancer cell lines *in vitro* | | | | | | | |
|---|---|---|---|---|---|---|---|
| | MCF-wt (µM) | | MDA 468 (µM) | | MCF7-Adr (µM) | | |
| Compound | A | B | A | B | A | B | C |
| CJM126 | 0.003 | - | 0.0016 | - | - | - | >1 |
| DF140 | 0.11 | - | - | >10 | - | - | 10,30 |
| DF161 | 0.001 | 55.9 | 0.0007 | 2.84 | - | 84.8 | 3,10,30 |
| 93002 | 0.38 | - | - | 7.64 | - | - | 3,10 |
| 93003 | 0.0046 | - | 0.0024 | - | - | - | 3,10 |
| 93004 | 0.142 | - | 0.003 | - | - | - | 1,3,10 |
| CJM129 | - | >10 | NT | NT | Inactive | | |
| DF126-126 | - | >10 | NT | NT | Inactive | | |
| 93005 | 0.063 | - | 0.0022 | NT | 0.0465 | - | 3,10 |
| 93006 | 0.867 | - | NT | NT | - | - | 3,10 |
| 93007 | - | 2.77 | NT | NT | - | - | 1,3,10 |
| 93008 | 0.160 | - | NT | NT | - | - | 3,10 |
| DF162Eb | - | 49.65 | - | 16.3 | - | 13.9 | - |
| DF162D | - | 19.3 | 0.0035 | - | - | 20.9 | - |
| DF68D | - | 22.3 | - | 19.3 | Inactive | | |
| A IC₅₀ at sub-micromolar concentrations B IC₅₀ at >micromolar concentrations C Concentrations which stimulate cell growth NT Not tested | | | | | | | |

**Table 4**

| *In vivo* activity of CJM 126 against MCF-7 mammary carcinoma | | | | | | | | | |
|---|---|---|---|---|---|---|---|---|---|
| Compound | Route | Dose mg/kg/day | BWC (%) | Relative tumour volume (T/C%) | | | | | |
| | | | | d9 | d13 | d20 | d27 | d35 | d43 |
| CJM 126 | i.p. | 120 | -13 | 84 | 47* | 38* | 27* | 27* | 43* |
| Mitoxantrone | i.v. | 10 | -10 | 52 | 32* | 38* | 26* | 29* | 29* |
| Mice: Bln. NMRI - nu/nu (female) Tumour: MCF-7 implanted s.c. supplemented with oestradiol Treatment schecule: qd 6, 13, 20 by indicated route. BWC: Body weight change relative to saline treated animals Control: Saline | | | | | | | | | |

| | | | | | | | | | |
|---|---|---|---|---|---|---|---|---|---|
| *Statistically significant (p<0.05) | | | | | | | | | |

**Table 5**

| *In vivo* activity of CJM 126 and CJM 129 against BO mammary carcinoma | | | | | | | | |
|---|---|---|---|---|---|---|---|---|
| Compound | Route | Dose (mg/kg/day) | BWC | Relative tumour volume (T/C%) | | | | |
| | | | | d33 | d41 | d47 | d55 | d61 |
| CJM 126 | i.p. | 100 | -7 | 137 | 77 | 87 | 68 | 52 |
| CJM 126 | i.p. | 10 | -4 | 107 | 74 | 55 | 70 | 52 |
| CJM 126 | i.p. | 1 | -5 | 173 | 72 | 25* | 67 | 41* |
| CJM 129 | i.p. | 200 | -6 | 78* | 45* | 32* | 33* | 36* |
| CJM 129 | i.p. | 20 | -3 | 88 | 77 | 54 | 73 | 48* |
| CJM 129 | i.p. | 2 | 2 | 127 | 91 | 94 | 95 | 78 |
| Vincristine | i.p. | 1 | -6 | 142 | 62 | 61 | 61 | 57 |
| Cyclophosphamide | i.p. | 150 | -4 | 117 | 27* | 29* | 19* | 5* |
| Mitoxantrone | i.v. | 10 | -5 | 58* | 26* | 6* | 7* | 4* |
| Treatment schedule: for CJM 126 and CJM 129 qd 27,34,41; for vincristine, cyclophosphamide and mitoxantrone qd 27. CJM 129 is 2-(3-aminophenyl)benzothiazole For other details: see footnotes to Table 4 | | | | | | | | |

**Table 6**

| Activity of CJM 126 against human MDA 468 tumour *in vivo*. | | | | |
|---|---|---|---|---|
| | Cross section tumour area (mm) Group | | | |
| Day | Control | .001mg/kg | .01mg/kg | 0.1mg/kg |
| 5 | 95.28 + 4.61 | 85.54 + 19.44 | 120.78 + 5.83 | 97.75 +9.55 |
| 12 | 62.88 + 8.10 | 52.93 + 2.65 | 71.65 + 11.67 | 71.07 +7.2 |
| 15 | 86.02 + 8.65 | 67.81 +12.92 | 78.21 + 6.69 | 82.17 + 1058 |
| 19 | 118.35 + 37.1 | 64.8 + 17.35 | 98.03 + 33.51 | 88.84 + 3.69 |
| 22 | 119.67 + 19.05 | 75.12 + 20.05 | 117.02 + 42.1 | 105.66 + 5.49 |
| 26 | 130.97 + 20.61 | 81.53 + 26.16 | 131.58 + 23.41 | 124.55 + 7.90 |
| 29 | 170.99 + 51.67 | 114.65 + 3457 | 164.90 + 37.18 | 182.15 + 14.79 |
| 32 | 191.0 + 48,36 | 129.06 + 32.08 | 191.68 + 45.65 | 196.66 + 18.65 |

| | Tumour weight (g) | | | |
|---|---|---|---|---|
| | .31 +.09 | .19 + .08 | .29 + .15 | .30 + .07 |

## Claims

1. A compound that is a benzazole compound represented by the structural formula I below, or a pharmaceutically acceptable salt thereof, 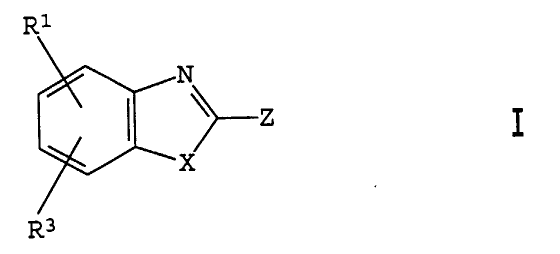 characterised in that
X is S or O;
R¹ and R³ are each independently hydrogen, alkyl, hydroxyl, alkoxy or aralkoxy; and
Z is a group represented by the structural formula II below 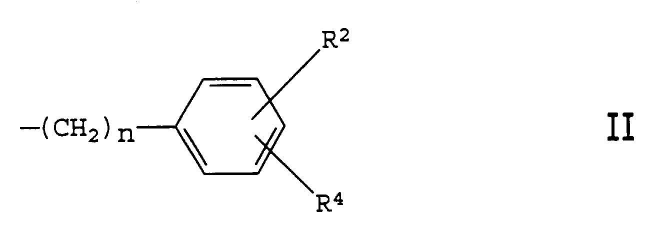 where
n = 0 or 1,
R² is hydrogen, NH₂, NO₂, N₃, halogen or an alkyl or substituted alkyl oxysulphonyl group; and
R⁴ is hydrogen, NO₂, N₃, pyrrolidino, piperidino, morpholino or NR⁵R⁶ where
R⁵ and R⁶ each represent hydrogen or alkyl;
with the proviso that when R⁴ is hydrogen R² is selected from NH₂, NO₂ and N₃, and when one of R² and R⁴ is hydrogen and the other is -NH₂ neither R¹ nor R³ is 6-alkyl,
and with the further proviso that alkyl groups when present as such in the compound or as a moiety in other groups such as alkoxy are each composed of less than 6 carbon atoms,
said compound being for use in therapy.

2. A compound as claimed in Claim 1 for use as an active therapeutic substance further characterised in that at least one of R² and R⁴ is an amino or a substituted amino group.

3. A 2-arylbenzazole compound as claimed in Claim 1 for use as a therapeutic agent wherein Z is

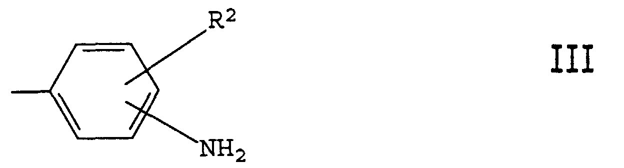

4. A compound as claimed in Claim 3 for use as an active therapeutic substance wherein R² is 2-Cl.

5. A compound as claimed in any of Claims 1 to 4 for use as an active therapeutic substance wherein R¹ is hydrogen and R³ is alkoxy or benzyloxy.

6. A compound as claimed in any of Claims 1 to 5 further characterised by at least one of the following features:
(a) at least some alkyl groups when present as such or as a moiety in other groups such as alkoxy are methyl or ethyl;
(b) halo substituents, when present, are selected from fluorine and chlorine;
(c) R² is hydrogen or 2-halogen, and R⁴ is amino.

7. A 2-arylbenzazole compound as claimed in any of the preceding claims for use as an active therapeutic substance, said compound being a substituted 2-phenylbenzothiazole compound.

8. A compound as claimed in Claim 1 for use as an active therapeutic substance, said compound being a substituted 2-phenylbenzazole compound characterised in that the combination of substituents R¹, R², R³, R⁴ and X is selected from the following combinations:

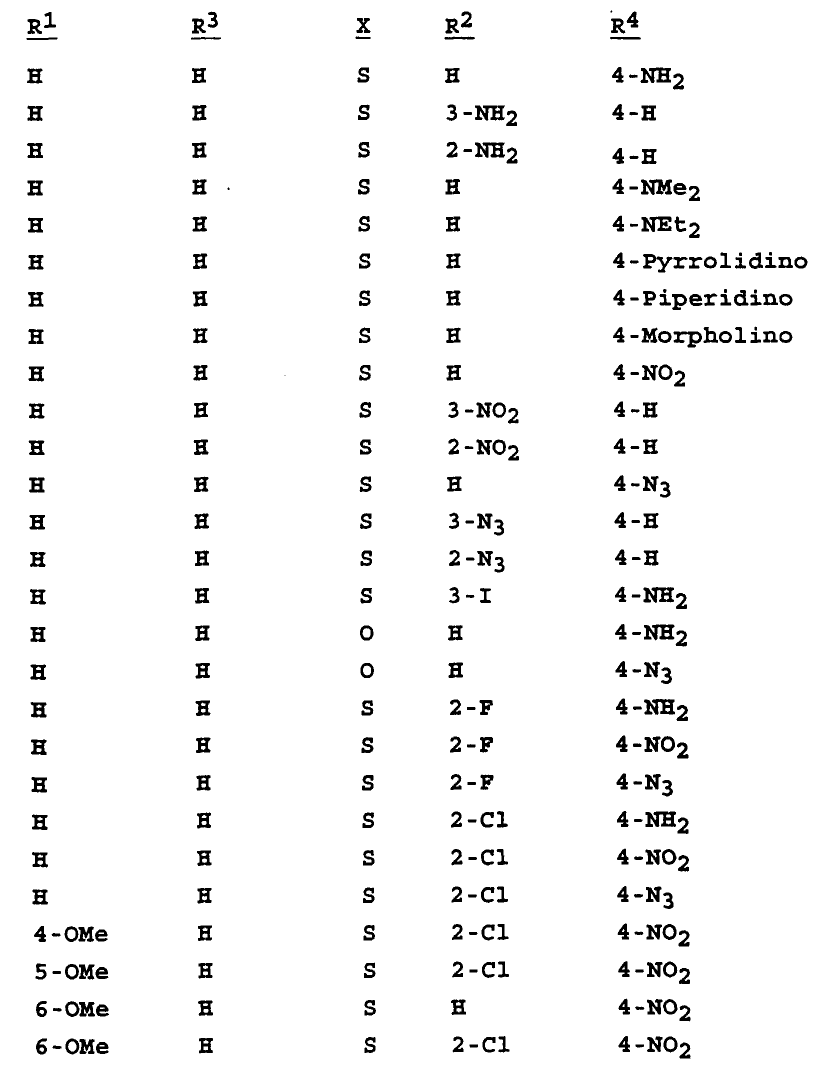

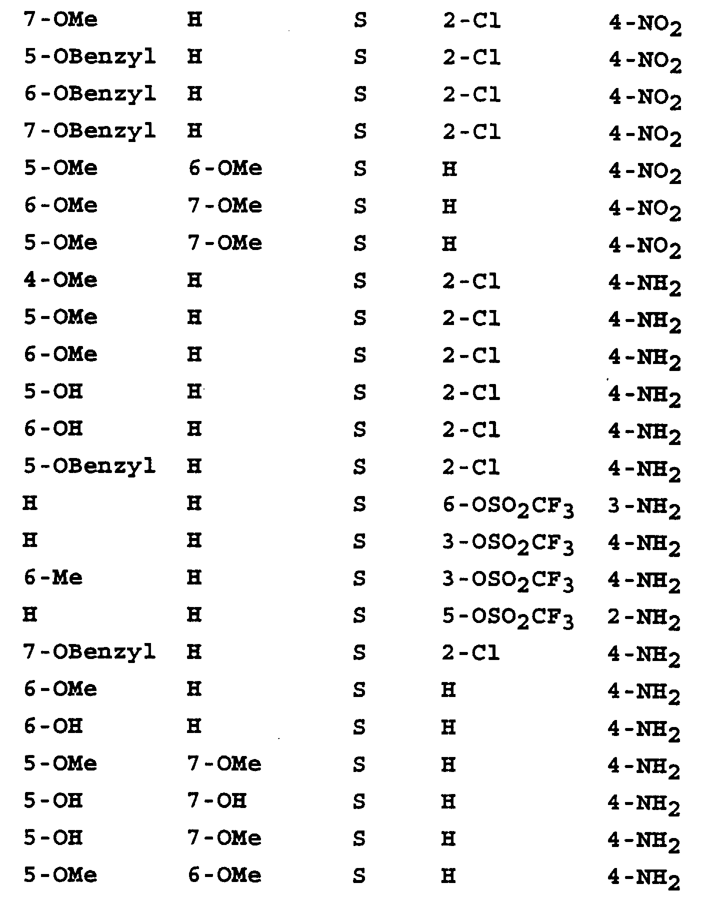

9. 2-(4-aminophenyl)benzothiazole for use as an active therapeutic substance or a pharmaceutically acceptable salt thereof.

10. A compound as claimed in Claim 1 for use as an active therapeutic substance, said compound being one of the following:
(1) 2-(4-Dimethylaminophenyl)benzothiazole
(2) 2-(4-Diethylaminophenyl)benzothiazole
(3) 2-(2-Aminophenyl)benzothiazole
(4) 2-(2-Fluorophenyl)benzothiazole
(5) 2-(4-Aminobenzyl)benzothiazole
(6) 2-[4-(Pyrrolidin-1-yl)phenyl]benzothiazole
(7) 2-(3-Nitrophenyl)benzothiazole
(8) 2-(2-Chloro-4-nitrophenyl)benzothiazole
(9) 6-Methoxy-2-(4-nitrophenyl)benzothiazole
(10) 2-(2-Chloro-4-nitrophenyl)-6-methoxybenzothiazole
(11) 2-(2-Chloro-4-nitrophenyl)-7-methoxybenzothiazole
(12) 2-(2-Chloro-4-nitrophenyl)-4-methoxybenzothiazole
(13) 2-(3-Aminophenyl)benzothiazole
(14) 2-(4-Amino-2-chlorophenyl)benzothiazole
(15) 2-(4-Amino-2-chlorophenyl)-4-methoxybenzothiazole
(16) 2-(4-Amino-2-chlorophenyl)-5-methoxybenzothiazole
(17) 2-(4-Amino-2-chlorophenyl)-6-methoxybenzothiazole
(18) 2-(4-Amino-2-chlorophenyl)-7-methoxybenzothiazole
(19) 2-(4-Azidophenyl)benzothiazole
(20) 2-[4-(Morpholin-4-yl)phenyl]benzothiazole
(21) 2-[4-(Piperidin-1-yl)phenyl]benzothiazole
(22) Ethanesulphonic acid salt of 2-(4-aminophenyl)-benzothiazole
(23) Dihydrochloride salt of 2-(4-aminophenyl)benzothiazole
(24) 2-(4-Aminophenyl)benzothiazole methanesulphonic acid salt

11. A compound as claimed in any of the preceding claims for use as an active therapeutic substance characterised in that it is an acid addition salt derived from an acid selected from the group comprising: hydrochloric, hydrobromic, sulphuric, nitric, phosphoric, maleic, salicylic, p-toluenesulphonic, tartaric, citric, lactobionic, formic, malonic, pantothenic, succinic, naphthalene-2-sulphonic, benzenesulphonic, methanesulphonic and ethanesulphonic.

12. A process for preparation of a compound having the general structure defined in Claim 2 wherein R² and/or R⁴ is amino, said process being characterised by the fact that it comprises the steps of preparing the corresponding nitro substituted compound, i.e. the compound with the same structural formula except that R² and/or R⁴ is a nitro group, and then reducing said nitro group(s) to amino.

13. A compound as claimed in any one of Claims 1 to 11 for therapeutic use in treating breast cancer in mammals.

14. A pharmaceutical formulation for medical use comprising, as the active compound, a compound as claimed in any one of Claims 1 to 11 together with a pharmaceutically acceptable carrier therefor.

15. A medical preparation containing a therapeutically effective non-toxic amount of a compound as claimed in any one of Claims 1 to 11 and a pharmaceutically inert excipient.

16. A pharmaceutical preparation in unit dosage unit form for administration to obtain a therapeutic effect as an antitumour agent in treating mammals, said preparation comprising, per dosage unit, a therapeutically-effective non-toxic amount of a compound as set forth in any one of Claims 1 to 11.

17. Use of a compound as claimed in any one of Claims 1 to 11 for the manufacture of a medical preparation for the treatment of tumours in mammals.

18. Use as claimed in Claim 17 wherein the medical preparation is for inhibiting the growth or proliferation of human breast cancer cells.

19. A method of manufacture of a medical preparation for the treatment of tumours in mammals comprising admixing a compound of any one of Claims 1 to 11 with a pharmaceutically acceptable carrier therefor.

20. A method as claimed in Claim 19 wherein the medical preparation is for inhibiting the growth or proliferation of human breast cancer cells.

## Patentansprüche

1. Verbindung, nämlich eine Benzazol-Verbindung, dargestellt durch die nachstehende Strukturformel I, oder deren pharmazeutisch verträgliches Salz, 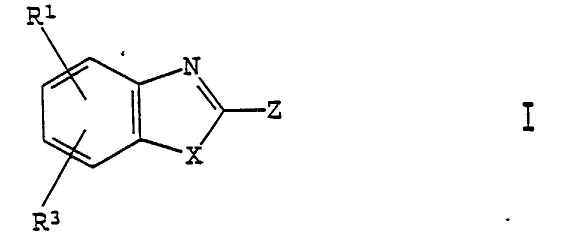 welche dadurch gekennzeichnet, daß
X die Elemente S oder O bedeutet;
R¹ und R³ jeweils unabhängig voneinander für Wasserstoff, Alkyl, Hydroxyl, Alkoxy oder Aralkoxy stehen; und
Z eine durch die nachstehende Strukturformel II 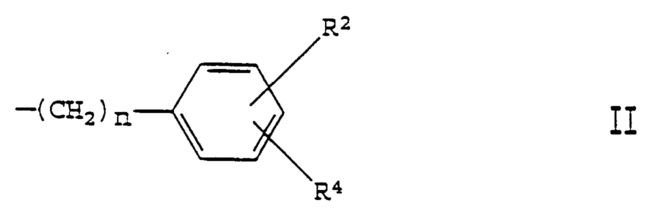 dargestellte Gruppe ist,
wobei
n = 0 oder 1,
R² Wasserstoff NH₂, NO₂, N₃, Halogen oder ein Alkyl oder eine substituierte Alkyloxysulfonyl-Gruppe ist; und
R⁴ Wasserstoff NO₂, N₃, Pyrrolidino-, Piperidino-, Morpholino-Gruppe oder NR⁵R⁶ ist, wobei
R⁵ und R⁶ jeweils Wasserstoff oder Alkyl bedeuten;
mit der Maßgabe, daß, sofern R⁴ Wasserstoff ist, R² ausgewählt wird aus NH₂, NO₂ und N₃, und, wenn eines der R² und R⁴ Wasserstoff ist und das andere -NH₂ ist, weder R¹ noch R³ ein 6-Alkyl ist,
und mit der weiteren Maßgabe, daß Alkyl-Gruppen, wenn sie als solche in der Verbindung oder als Teil in anderen Gruppen, wie zum Beispiel Alkoxy, vorliegen, aus weniger als 6 Kohlenstoffatomen zusammengesetzt sind,
wobei die Verbindung zur Verwendung in der Therapie dient.

2. Verbindung nach Anspruch 1 zur Verwendung als aktive, therapeutische Substanz ferner dadurch gekennzeichnet, daß wenigstens einer von R² oder R⁴ eine Amino- oder eine substituierte Amino-Gruppe ist.

3. 2-Arylbenzazol-Verbindung nach Anspruch 1 zu Verwendung als Therapiemittel, dadurch gekennzeichnet, daß Z umfaßt

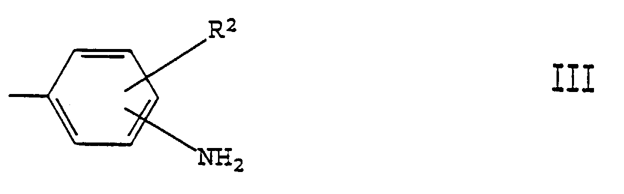

4. Verbindung nach Anspruch 3 zur Verwendung als aktive, therapeutische Substanz dadurch gekennzeichnet, daß R² 2-Cl ist.

5. Verbindung nach einem der Ansprüche 1 bis 4 zur Verwendung als aktive, therapeutische Substanz, dadurch gekennzeichnet, daß R¹ Wasserstoff und R³ Alkoxy oder Benzyloxy ist.

6. Verbindung nach einem der Ansprüche 1 bis 5, ferner gekennzeichnet durch wenigstens eines der nachfolgenden Merkmale:
(a) wenigstens einige Alkyl-Gruppen, wenn diese als solche oder als Teil in anderen Gruppen, wie zum Beispiel Alkoxy vorliegen, sind Methyl oder Ethyl;
(b) Halogensubstituenten, sofern vorliegend, werden ausgewählt aus Fluor und Chlor;
(c) R² ist Wasserstoff oder 2-Halogen, und R⁴ ist eine Amino-Gruppe.

7. 2-Arylbenzol-Verbindung nach einem der vorhergehenden Ansprüche zur Verwendung als aktive, therapeutische Substanz dadurch gekennzeichnet, daß die Verbindung eine substituierte 2-Phenylbenzothiazol-Verbindung ist.

8. Verbindung nach Anspruch 1, zur Verwendung als aktive, therapeutische Substanz, wobei die Verbindung eine substituierte 2-Phenylbenzazol-Verbindung ist, dadurch gekennzeichnet, daß die Kombination der Substituenten R¹, R², R³, R⁴ und X aus den nachfolgenden Kombinationen ausgewählt ist:

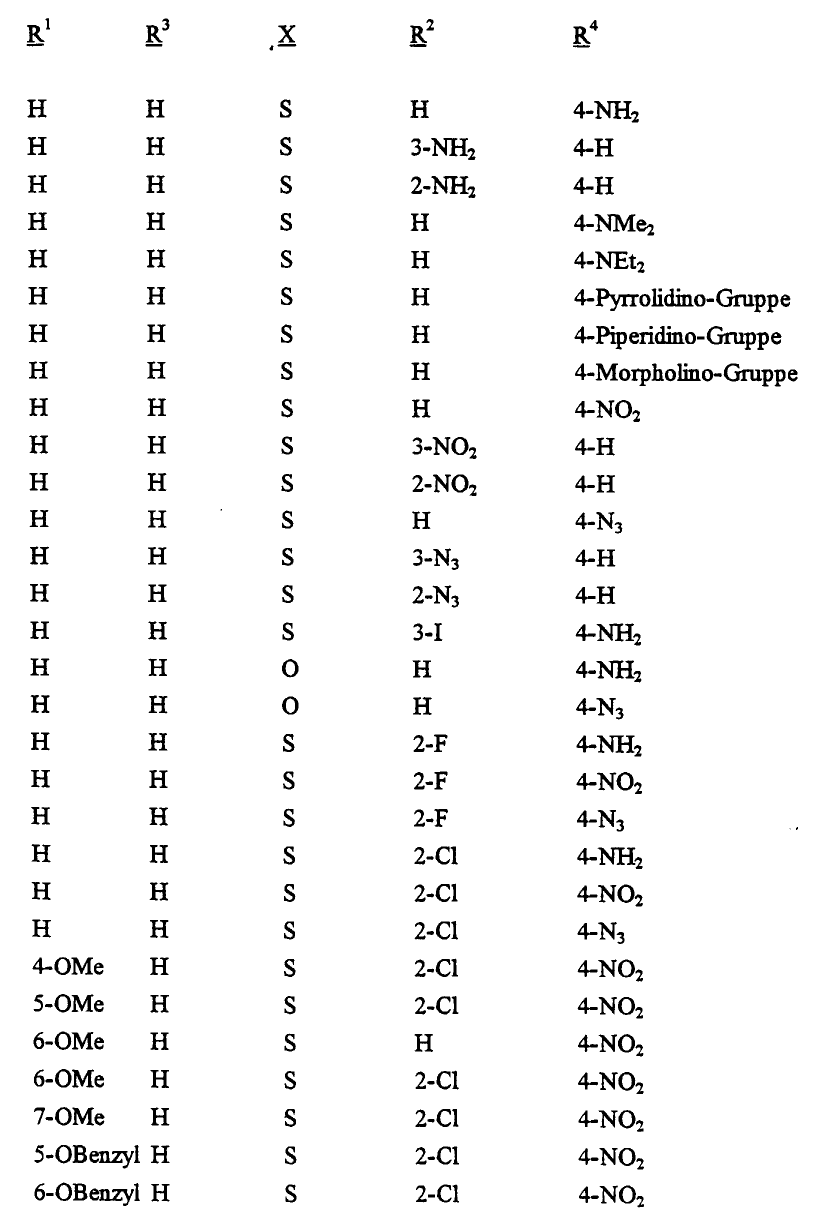

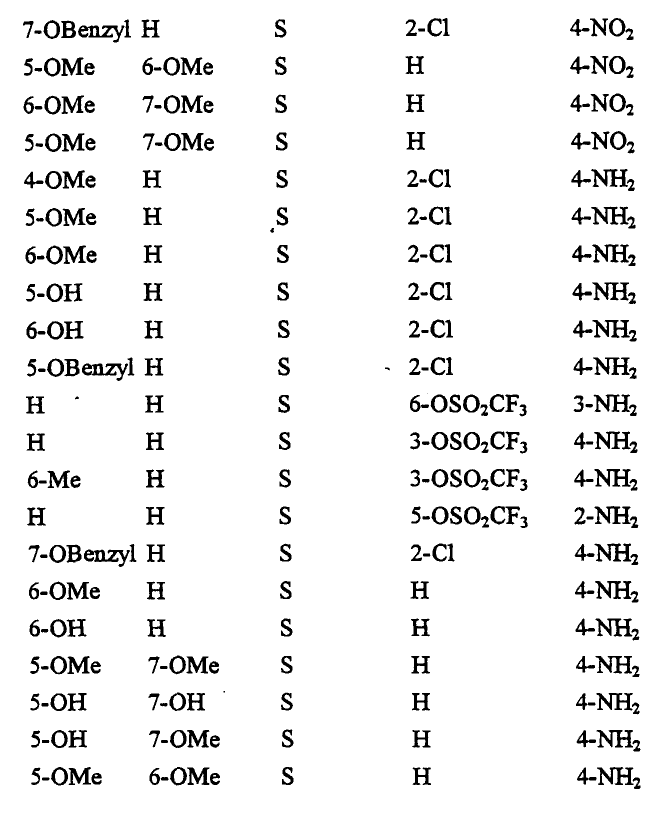

9. 2-(4-Aminophenyl)benzothiazol zur Verwendung als aktive, therapeutische Substanz oder als dessen pharmazeutisch verträgliches Salz.

10. Verbindung nach Anspruch 1 zur Verwendung als aktive, therapeutische Substanz, gekennzeichnet durch eine der folgenden Verbindungen:
(1) 2-(4-Dimethylaminophenyl)benzothiazol
(2) 2-(4-Diethylaminophenyl)benzothiazol
(3) 2-(2-Aminophenyl)benzothiazol
(4) 2-(2-Fluorphenyl)benzothiazol
(5) 2-(4-Aminobenzyl)benzothiazol
(6) 2-[4-(Pyrrolidin-1-yl)phenyl]benzothiazol
(7) 2-(3-Nitrophenyl)benzothiazol
(8) 2-(2-Chlor-4-nitrophenyl)benzothiazol
(9) 6-Methoxy-2-(4-nitrophenyl)benzothiazol
(10) 2-(2-Chlor-4-nitrophenyl)-6-methoxybenzothiazol
(11) 2-(2-Chlor-4-nitrophenyl)-7-methoxybenzothiazol
(12) 2-(2-Chlor-4-nitrophenyl)-4-methoxybenzothiazol
(13) 2-(3-Aminophenyl)benzothiazol
(14) 2-(4-Amino-2-chlorphenyl)benzothiazol
(15) 2-(4-Amino-2-chlorphenyl)-4-methoxybenzothiazol
(16) 2-(4-Amino-2-chlorphenyl)-5-methoxybenzothiazol
(17) 2-(4-Amino-2-chlorphenyl)-6-methoxybenzothiazol
(18) 2-(4-Amino-2-chlorphenyl)-7-methoxybenzothiazol
(19) 2-(4-Azidophenyl)benzothiazol
(20) 2-[4-(Morpholin-4-yl)phenyl]benzothiazol
(21) 2-[4-(Piperidin-1-yl)phenyl]benzothiazol
(22) Ethansulfonsäursalz von 2-(4-Aminophenyl)benzothiazol
(23) Dihydrochloridsalz von 2-(4-Aminophenyl)benzolthiazol
(24) 2-(4-Aminophenyl)benzothiazolmethansulfonsäuresalz

11. Verbindung nach einem der vorhergehenden Ansprüche zur Verwendung als aktive, therapeutische Substanz, dadurch gekennzeichnet, daß sie ein Säureadditionssalz ist, das von einer Säure erhalten wird, die aus der nachfolgenden Gruppe ausgewählt ist: Salzsäure, Hydrobromsäure, Schwefelsäure, Salpetersäure, Phosphorsäure, Maleinsäure, Salicylsäure, p-Toluolsulfonsäure, Weinsäure, Zitronensäure, Lactobionsäure, Ameiensäure, Malonsäure, Pantothensäure, Succinsäure, Naphthalin-2-sulphonsäure, Benzolsulphonsäure, Methansulfonsäure und Ethansulfonsäure.

12. Verfahren zum Herstellen einer Verbindung mit der in Anspruch 2 angegbenen allgemeinen Struktur, wobei R² und/oder R⁴ eine Amino-Gruppe ist, dadurch gekennzeichnet, daß es die Schritte Herstellen der entsprechenden, nitrosubstituierten Verbindung, d. h. der Verbindung mit derselben Strukturformel, ausgenommen, daß R² und/oder R⁴ eine Nitro-Gruppe ist, und anschließendes Reduzieren der Nitro-Gruppe(n) zu Amino-Gruppen umfaßt.

13. Verbindung nach einem der Ansprüche 1 bis 11 zur therapeutischen Verwendung bei der Behandlung von Brustkrebs bei Säugern.

14. Pharmazeutische Zubereitung für die medizinische Verwendung, umfassend als aktive Verbindung eine Verbindung nach einem der Ansprüche 1 bis 11 zusammen mit einem pharmazeutisch verträglichen Trägerstoff.

15. Medizinisches Arzneimittel enthaltend eine therapeutisch wirksame, nichttoxische Menge einer Verbindung nach einem der Ansprüche 1 bis 11 und ein pharmazeutisch inertes Trägermaterial.

16. Pharmazeutisches Arzneimittel in Einheitsdosierungsform zur Verabreichung zum Herbeiführen eines therapeutischen Effektes als Antitumormittel bei der Behandlung von Säugern, wobei das Arzneimittel pro Dosierungseinheit eine therapeutisch wirksame, nichttoxische Menge einer Verbindung nach einem der Ansprüche 1 bis 11 aufweist.

17. Verwendung einer Verbindung nach einem der Ansprüche 1 bis 11 zur Herstellung eines medizinischen Arzneimittels für die Behandlung von Tumoren bei Säugern.

18. Verwendung nach Anspruch 17, dadurch gekennzeichnet, daß das medizinische Arzneimittel zum Hemmen des Wachstums oder der Prolifieration von menschlichen Brustkrebszellen dient.

19. Verfahren zum Herstellen eines medizinischen Arzneimittels für die Behandlung von Tumoren in Säugern, umfassend Mischen einer Verbindung nach einem der Ansprüche 1 bis 11 mit einem pharmazeutisch verträglichen Trägerstoff.

20. Verfahren nach Anspruch 19, dadurch gekennzeichnet, daß das medizinische Arzneimittel zum Verhindern des Wachstums oder der Prolideration von menschlichen Brustkrebszellen dient.

## Revendications

1. Composé qui est un composé de benzazole représenté par la formule développée I ci-dessous, ou sel pharmaceutiquement acceptable de celui-ci, 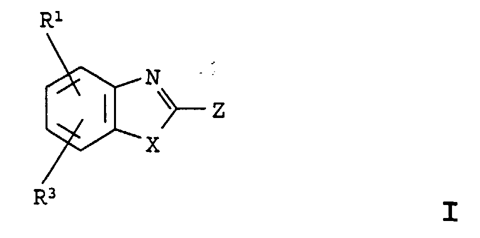 caractérisé en ce que
X représente S ou O;
R¹ et R³ représentent chacun indépendamment un atome d'hydrogène, un groupe alkyle, hydroxyle, alkoxy ou aralkoxy; et
Z est un groupe représenté par la formule développée II ci-dessous : 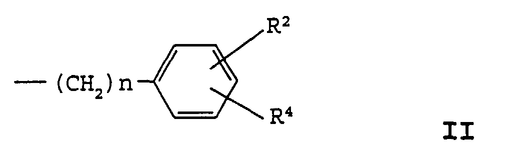 dans laquelle
n = 0 ou 1,
R² représente un atome d'hydrogène, NH₂, NO₂, N₃, un atome d'halogène ou un groupe alkyle ou alkyloxysulfonyle substitué; et
R⁴ représente un atome d'hydrogène, NO₂, N₃, un groupe pyrrolidino, pipéridino, morpholino ou NR⁵R⁶ dans lequel
R⁵ et R⁶ représentent chacun indépendamment un atome d'hydrogène ou un groupe alkyle;
à la condition que lorsque R⁴ est un atome d'hydrogène R² soit choisi parmi NH₂, NO₂ et N₃, et
lorsque un entre R² et R⁴ est un atome d'hydrogène et que l'autre représente -NH₂ ni R¹ ni R³ ne représente un groupe 6-alkyle,
et à la condition supplémentaire que les groupes alkyle lorsqu'ils sont présents comme tels dans le composé ou comme fragment dans d'autres groupes tels que alkoxy soient chacun composé de moins de 6 atomes de carbone,
ledit composé étant à usage thérapeutique.

2. Composé selon la revendication 1, à utiliser comme principe actif thérapeutique caractérisé en outre en ce qu'au moins un de R² et R⁴ est un groupe amino ou amino substitué.

3. Composé de 2-arylbenzazole selon la revendication 1 à utiliser comme agent thérapeutique dans lequel Z représente :

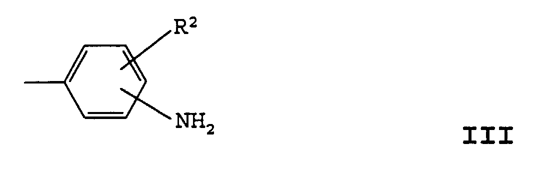

4. Composé selon la revendication 3 à utiliser comme principe actif thérapeutique dans lequel R² représente le groupe 2-Cl.

5. Composé selon l'une quelconque des revendications 1 à 4 à utiliser comme principe actif thérapeutique dans lequel R¹ représente un atome d'hydrogène et R³ représente un groupe alkoxy ou benzyloxy.

6. Composé selon l'une quelconque des revendications 1 à 5 caractérisé en outre par au moins un des aspects suivants :
(a) au moins certains groupes alkyle lorsqu'ils sont présents comme tels ou comme fragment dans d'autres groupes tels que alkoxy sont des groupes méthyle ou éthyle;
(b) les substituants halogènes, lorsqu'ils sont présents, sont choisis entre le fluor et le chlore;
(c) R² représente un atome d'hydrogène ou un groupe 2-halogène, et R⁴ est un groupe amino.

7. Composé de 2-arylbenzazole selon l'une quelconque des revendications précédentes à utiliser comme principe actif thérapeutique, ledit composé étant un composé de 2-phénylbenzothiazole substitué.

8. Composé selon la revendication 1 à utiliser comme principe actif thérapeutique, ledit composé étant un composé de 2-phénylbenzazole substitué caractérisé en ce que l'association des substituants R¹, R², R³, R⁴ et X est choisie parmi les associations suivantes :

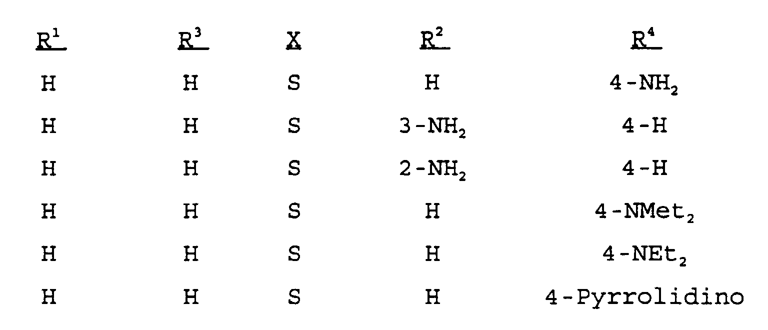

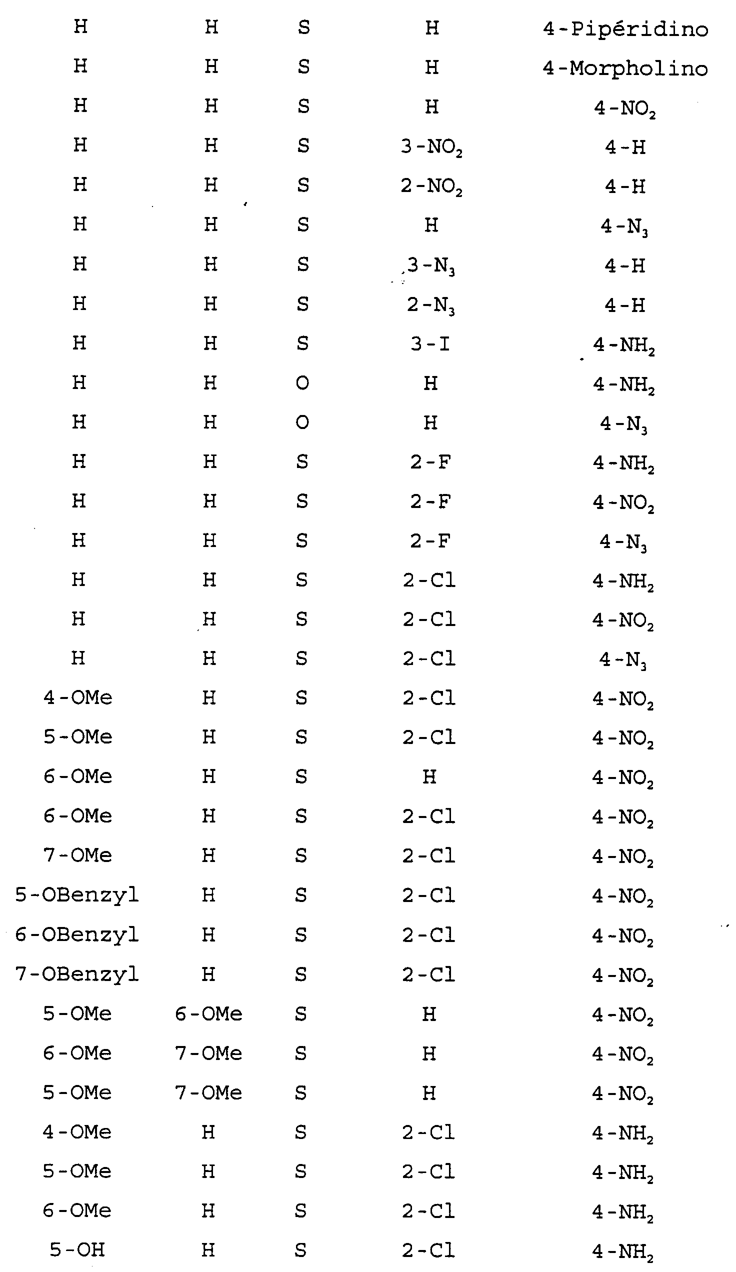

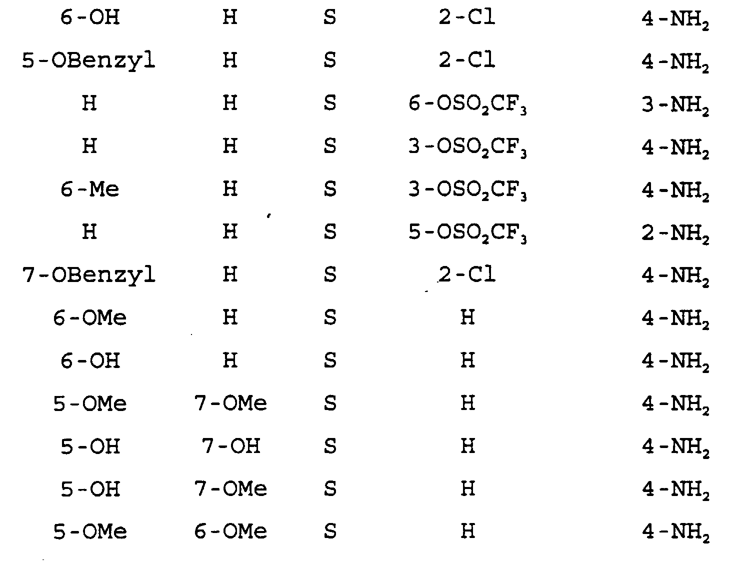

9. 2-(4-aminophényl)benzothiazole à utiliser comme principe actif thérapeutique ou sel pharmaceutiquement acceptable de celui-ci.

10. Composé selon la revendication 1 à usage de principe actif thérapeutique, ledit composé étant un des suivants :
(1) 2-(4-Diméthylaminophényl)benzothiazole
(2) 2-(4-Diéthylaminophényl)benzothiazole
(3) 2-(2-Aminophényl)benzothiazole
(4) 2-(2-Fluorophényl)benzothiazole
(5) 2-(4-Aminobenzyl)benzothiazole
(6) 2-[4-(Pyrrolidin-1-yl)phényl]benzothiazole
(7) 2-(3-Nitrophényl)benzothiazole
(8) 2-(2-Chloro-4-nitrophényl)benzothiazole
(9) 6-Méthoxy-2-(4-nitrophényl)benzothiazole
(10) 2-(2-Chloro-4-nitrophényl)-6-méthoxybenzothiazole
(11) 2-(2-Chloro-4-nitrophényl)-7-méthoxybenzothiazole
(12) 2-(2-Chloro-4-nitrophényl)-4-méthoxybenzothiazole
(13) 2-(3-Aminophényl)benzothiazole
(14) 2-(4-Amino-2-chlorophényl)benzothiazole
(15) 2-(4-Amino-2-chlorophényl)-4-méthoxybenzothiazole
(16) 2-(4-Amino-2-chlorophényl)-5-méthoxybenzothiazole
(17) 2-(4-Amino-2-chlorophényl)-6-méthoxybenzothiazole
(18) 2-(4-Amino-2-chlorophényl)-7-méthoxybenzothiazole
(19) 2-(4-Azidophényl)benzothiazole
(20) 2-[4-(Morpholin-4-yl)phényl]benzothiazole
(21) 2-[4-(Pipéridin-1-yl)phényl]benzothiazole
(22) Sel d'acide éthanesulfonique de 2-(4-aminophényl)-benzothiazole
(23) Sel dichlorhydrate de 2-(4-aminophényl)benzothiazole
(24) Sel d'acide méthanesulfonique de 2-(4-aminophényl)-benzothiazole.

11. Composé selon l'une quelconque des revendications précédentes à usage de principe actif thérapeutique caractérisé en ce qu'il est un sel d'addition d'acide dérivé d'un acide choisi parmi le groupe comprenant : l'acide chlorhydrique, bromhydrique, sulfurique, nitrique, phosphorique, maléique, salicylique, p-toluènesulfonique, tartrique, citrique, lactobionique, formique, malonique, pantothénique, succinique, naphtalène-2-sulfonique, benzènesulfonique, méthanesulfonique et éthanesulfonique.

12. Procédé de préparation d'un composé ayant la structure générale définie dans la revendication 2 dans lequel R² et/ou R⁴ est un groupe amino, ledit procédé étant caractérisé par le fait qu'il comprend les étapes de préparation du composé nitro-substitué correspondant, c'est-à-dire le composé avec la même formule développée excepté que R² et/ou R⁴ est un groupe nitro, et ensuite la réduction dudit (desdits) groupe(s) nitro en amino.

13. Composé selon l'une quelconque des revendications 1 à 11 à usage thérapeutique dans le traitement du cancer du sein chez les mammifères.

14. Formulation pharmaceutique à usage médical comprenant, comme principe actif, un composé selon l'une quelconque des revendications 1 à 11 avec un vecteur pharmaceutiquement acceptable pour celui-ci.

15. Préparation médicale contenant une quantité thérapeutiquement efficace non toxique d'un composé selon l'une quelconque des revendications 1 à 11 et un excipient pharmaceutiquement inerte.

16. Préparation pharmaceutique sous forme d'unités de dosage à l'unité à administrer pour obtenir un effet thérapeutique comme agent antitumoral dans le traitement de mammifères, ladite préparation comprenant, par unité de dosage, une quantité thérapeutiquement efficace non toxique d'un composé tel que présenté dans l'une quelconque des revendications 1 à 11.

17. Utilisation d'un composé selon l'une quelconque des revendications 1 à 11 pour la fabrication d'une préparation médicale pour le traitement de tumeurs chez les mammifères.

18. Utilisation selon la revendication 17 dans laquelle la préparation médicale sert à inhiber la croissance ou la prolifération de cellules cancéreuses du sein humaines.

19. Procédé de fabrication d'une préparation médicale pour le traitement de tumeurs chez les mammifères comprenant le mélange d'un composé selon l'une quelconque des revendications 1 à 11 avec un vecteur pharmaceutiquement acceptable pour celui-ci.

20. Procédé selon la revendication 19 dans lequel la préparation médicale sert à inhiber la croissance ou la prolifération de cellules cancéreuses du sein humaines.
